# EUROPÄISCHE PATENTANMELDUNG

(11) **EP 3 797 585 A1**
(43) Veröffentlichungstag der Anmeldung: **31.03.2021**
(21) Anmeldenummer: 19200556.9
(22) Anmeldetag: 30.09.2019
(51) Int. Cl.: A01K 27/00

(54) **VORRICHTUNG ZUR BESTIMMUNG EINES PARAMETERS DER FORTBEWEGUNG VON TIEREN**

(71) Anmelder: 4DVets AG, 4402 Frenkendorf (CH)
(72) Erfinder: SCHKOMMODAU, Erik, 4410 Liestal (CH); MONNIER, Patrick Blättler, 4437 Waldenburg (CH); PISON, Daniela, 7000 Chur (CH)
(74) Vertreter: Kohler Schmid Möbus Patentanwälte

(57) **Zusammenfassung**

Eine Vorrichtung (1) zur Bestimmung eines Parameters der Fortbewegung von Tieren, nämlich von vierbeinigen Säugetieren, insbesondere von Hunden, Pferden und/oder Kamelen, umfasst wenigstens einen Marker (7) mit einem Tracker (36), und/oder wenigstens einen Sensor (8) sowie eine Auswerteeinheit (37). Der Marker (7) und/oder der Sensor (8) ist mit dem Körper des Tieres verbindbar, indem der Marker (7) und/oder der Sensor (8) an einer zum Bekleiden des Tieres vorgesehenen Jacke (2) angebracht ist. Die Jacke (2) weist wenigstens einen Rumpfteil (3) und einen Beinteil (4) auf, wobei der Rumpfteil (3) zur Aufnahme des Rumpfes des Tieres und der Beinteil (4) zur Aufnahme einer Extremität des Tieres ausgebildet sind. Der Marker (7) ist an der Jacke (2) derart angeordnet, dass der Marker (7) an dem mit der Jacke (2) bekleideten Tier mit einer definierten räumlichen Zuordnung zu einer dem Marker (7) zugeordneten parameterrelevanten Körperstelle des Tieres angeordnet ist. Ergänzend oder alternativ ist der Sensor (8) an der Jacke (2) derart angeordnet, dass der Sensor (8) an dem mit der Jacke (2) bekleideten Tier mit einer definierten räumlichen Zuordnung zu einer dem Sensor (8) zugeordneten parameterrelevanten Körperstelle des Tieres angeordnet ist. Mittels der Auswerteeinheit (37) wird anhand einer von dem Tracker (36) erfassten Bewegung der dem Marker (7) zugeordneten parameterrelevanten Körperstelle und/oder anhand einer von dem Sensor (8) erfassten Bewegung der dem Sensor (8) zugeordneten parameterrelevanten Körperstelle ein Parameter der Fortbewegung bestimmt.

Eine Jacke (2) ist zur Verwendung als Teil der Vorrichtung (1) ausgebildet.

## Beschreibung

Die Erfindung betrifft eine Vorrichtung zur Bestimmung eines Parameters der Fortbewegung von Tieren, nämlich von vierbeinigen Säugetieren, insbesondere von Hunden, Pferden und/oder Kamelen,
- mit wenigstens einem Marker und einem für den Marker vorgesehenen Tracker, wobei der Marker mit einer definierten räumlichen Zuordnung zu einer dem Marker zugeordneten parameterrelevanten Körperstelle des Tieres mit dem Körper des Tieres verbindbar ist und mittels des Markers von dem Tracker eine mit der Fortbewegung des Tieres verbundene Bewegung der dem Marker zugeordneten parameterrelevanten Körperstelle detektierbar ist, wobei die mit der Fortbewegung des Tieres verbundene Bewegung der dem Marker zugeordneten parameterrelevanten Körperstelle für den Parameter der Fortbewegung spezifisch ist und/oder
- mit wenigstens einem Sensor, der mit einer definierten räumlichen Zuordnung zu einer dem Sensor zugeordneten parameterrelevanten Körperstelle des Tieres mit dem Körper des Tieres verbindbar ist und mittels dessen eine mit der Fortbewegung des Tieres verbundene Bewegung der dem Sensor zugeordneten parameterrelevanten Körperstelle detektierbar ist, wobei die mit der Fortbewegung des Tieres verbundene Bewegung der dem Sensor zugeordneten parameterrelevanten Körperstelle für den Parameter der Fortbewegung spezifisch ist, sowie
- mit einer Auswerteeinheit, mittels derer anhand der von dem Tracker erfassten Bewegung der dem Marker zugeordneten parameterrelevanten Körperstelle und/oder anhand der von dem Sensor erfassten Bewegung der dem Sensor zugeordneten parameterrelevanten Körperstelle der Parameter der Fortbewegung bestimmbar ist.

Die Erfindung betrifft des Weiteren eine Jacke zum Bekleiden eines Tieres, nämlich eines vierbeinigen Säugetieres, insbesondere eines Hundes, eines Pferdes und/oder eines Kamels, die wenigstens einen Rumpfteil und einen Beinteil aufweist, wobei der Rumpfteil zur Aufnahme des Rumpfes des Tieres und der Beinteil zur Aufnahme einer Extremität des Tieres ausgebildet sind.

Beispielsweise Hunde, Pferde und Kamele können aufgrund von neurologischen und orthopädischen Erkrankungen in ihrer Fortbewegung beeinträchtigt sein. Häufige orthopädische Erkrankungen bei Hunden sind neben Rückenerkrankungen Hüft- und Ellbogendysplasien, Osteochondrosen im Schultergelenk sowie arthrotische und sonstige pathologische Veränderungen an den Hüft- und den Kniegelenken.

Es ist üblich, zum Untersuchen des Gesundheitszustandes des Bewegungsapparates und gegebenenfalls zum Diagnostizieren von orthopädischen Erkrankungen von Tieren den Gang des betroffenen Tieres zu analysieren. Im Rahmen einer Ganganalyse werden Parameter der Fortbewegung des Tieres bestimmt, die eine Aussagekraft in Bezug auf das Vorliegen und gegebenenfalls den Schweregrad einer orthopädischen Erkrankung besitzen. Zu den insoweit relevanten Gangparametern zählen etwa bei Hunden unter anderem die Schrittlänge, die Rückenbewegung, die Bodenkontaktzeit der Pfoten sowie die Länge und die Dauer des Schrittzyklus einschließlich der Dauer der Standphase des Schrittzyklus und einschließlich der Länge und des Gelenkwinkels ("range of motion") sowie der Dauer der Schwingphase des Schrittzyklus.

Eine gattungsgemäße Vorrichtung, im Falle derer mit Hilfe von als optische Marker vorgesehenen Retroreflektoren und mit Hilfe eines mehrere Infrarotkameras umfassenden Trackers eine Ganganalyse bei Hunden durchgeführt wird, ist beschrieben in dem Artikel mit dem Titel "Pelvic limb kinematics in the dog with and without a stifle orthosis", erschienen in Veterinary Surgery 2017 vol. 64, Seiten 642 bis 652. Retroreflektierende Marker sind dabei im Bereich des rechten Hinterbeins des betreffenden Hundes mit doppelseitigem Klebeband und Cyanacrylatklebstoff auf die Haut des Hundes aufgeklebt. Weitere retroreflektierende Marker sind an einer Orthese angebracht, die dem Hund zeitweise an dem rechten Hinterbein angelegt wird. Sowohl die auf die Haut des Hundes aufgeklebten Marker als auch die an der Orthese angebrachten Marker befinden sich gegenüber den an dem Bein des Hundes vorgesehenen Gelenken in einer definierten Position. Der Hund bewegt sich im Schritt und im Trab jeweils zunächst ohne und danach mit angelegter Orthese entlang eines Parcours. Dabei werden mittels der Infrarotkameras die retroreflektierenden Marker erfasst. Rechnergestützt wird aus den erfassten Bewegungen der retroreflektierenden Marker der Einfluss der Orthese auf das Gangbild des Hundes, im Einzelnen auf die Bewegung der Gelenke an dem rechten Hinterbein des Hundes, ermittelt.

Eine gattungsgemäße Vorrichtung, die sich zur Durchführung von Ganganalysen bei Hunden Inertialsensoren bedient, ist bekannt aus dem Aufsatz mit dem Titel "Evaluation of an open source method for calculating physical activity in dogs from harness and collar based sensors", erschienen in BMC Veterinary Research (2017) 13:322. In diesem Fall werden Bewegungen von Hunden mit Hilfe von vier Inertialsensoren erfasst, von denen jeweils zwei an einem dem Hund umgelegten Halsband und an Gurten eines dem Hund angelegten Geschirrs befestigt sind.

Ausgehend von dem gattungsgemäßen Stand der Technik besteht die Aufgabe der vorliegenden Erfindung darin, die Reproduzierbarkeit der Erfassung von Parametern der Fortbewegung von Tieren, insbesondere die Reproduzierbarkeit der Erfassung von Gangparametern bei Hunden, Pferden und Kamelen, zu verbessern.

Erfindungsgemäß gelöst wird diese Aufgabe durch die Vorrichtung gemäß Patentanspruch 1 und durch die Jacke gemäß Patentanspruch 12.

Im Falle der Erfindung ist wenigstens ein zur Durchführung einer Fortbewegungsinsbesondere einer Ganganalyse dienender Marker und/oder wenigstens ein zur Durchführung einer Ganganalyse ausgebildeter Sensor an einer Jacke befestigt, welche dem zu untersuchenden Tier angezogen wird. Zumindest ein Bein, vorzugsweise sämtliche Beine des Tieres werden von jeweils einem Beinteil der Jacke aufgenommen, ein mit dem oder den Beinteilen verbundener Rumpfteil der Jacke ist für den Rumpf des Tieres vorgesehen. Die Marker und/oder die Sensoren sind an definierten Befestigungspunkten mit der Jacke fest oder lösbar verbunden. Als Marker kommen insbesondere herkömmliche optische Marker wie etwa retroreflektierende Marker oder Farbmarkierungen in Frage, die mit Hilfe von als Tracker vorgesehenen Kameras detektiert werden und die an der Jacke für die Kameras zugänglich fixiert sind. Als Sensoren können insbesondere Inertialsensoren üblicher Bauart, vorzugsweise in der Praxis bewährte IMU's, verwendet werden. In bevorzugter Ausgestaltung der Erfindung sind die Stromspeicher von Sensoren bei an der Jacke angebrachtem Sensor aufladbar. Die Auswertung der erfassten Bewegungen erfolgt in üblicher Weise rechnergestützt mit Hilfe einer für Analysen der vorliegenden Art gebräuchlichen Software.

Die Passform der Jacke ist an den Körper des Tieres derart angepasst, dass die genannten Teile der Jacke im Zusammenspiel die Jacke an dem mit der Jacke bekleideten Tier formschlüssig festlegen. Der Formschluss bewirkt, dass die Jacke an dem Tier eine definierte Position einnimmt und dabei gegen ein unerwünschtes Verrutschen gesichert ist. In bevorzugter Ausgestaltung der Erfindung ist zusätzlich die Passform wenigstens eines der Teile der Jacke derart gewählt, dass die Jacke an den zugeordneten Körperteil des Tieres mit einer vorzugsweise geringen Vorspannung angelegt ist. Das Material der Jacke kann entsprechend beschaffen sein. Denkbar sind insbesondere Jacken oder Jackenteile aus Stretchgewebe, die sich dem Körper des Tieres eng anliegend anpassen und auf diese Weise dafür sorgen, dass die Jacke an dem mit der Jacke bekleideten Tier definiert und ortsunveränderlich positioniert ist. Für die Jacke bevorzugt werden außerdem waschbare Materialien.

Mit der definierten und ortsunveränderlichen Anordnung der Jacke an dem mit der Jacke bekleideten Tier geht eine entsprechende Anordnung des wenigstens einen an der Jacke angebrachten Markers und/oder des wenigstens einen an der Jacke angebrachten Sensors einher. Dabei sind die Befestigungsstellen der Marker und der Sensoren an der Jacke derart gewählt, dass die Marker und Sensoren an dem mit der Jacke bekleideten Tier mit definierter räumlicher Zuordnung zu parameterrelevanten Körperstellen beziehungsweise Körperteilen, beispielsweise mit definierter räumlicher Zuordnung zu Teilen der Gliedmaßen und zu Gelenken des Tieres, positioniert sind.

Eine gute Reproduzierbarkeit der Bestimmung von Fortbewegungs-, insbesondere von Gangparametern gewährleistet die erfindungsgemäße Jacke dadurch, dass sie an ein und demselben Individuum bei zeitlich voneinander getrennten Fortbewegungs- beziehungsweise Ganganalysen, stets in gleicher Weise positioniert ist, sofern sich der Körperbau des untersuchten Individuums zwischenzeitlich nicht nennenswert verändert hat. Unter dieser Prämisse ergibt sich für sämtliche der zu unterschiedlichen Zeitpunkten durchgeführten Analysen eine einheitliche räumliche Zuordnung des oder der an der Jacke angebrachten Marker und/oder des oder der an der Jacke angebrachten Sensoren zu den jeweiligen parameterrelevanten Körperstellen des zu untersuchenden Tieres. In gleicher Weise können mit der Jacke an voneinander verschiedenen Individuen mit einem im Wesentlichen einheitlichen Körperbau miteinander vergleichbare Untersuchungsergebnisse gewonnen werden.

Etwa der Besitzer eines ausgewachsenen Hundes kann für diesen Hund eine auf dessen Körperbau abgestimmte Jacke vorhalten, die bei zeitlich gegeneinander versetzten Untersuchungen des Tieres miteinander vergleichbare Untersuchungsergebnisse liefert und die es dadurch beispielsweise ermöglicht, Erkenntnisse über den zeitlichen Verlauf einer orthopädischen Erkrankung des Tieres zu gewinnen. Insbesondere für Tierärzte und/oder für die tiermedizinische Forschung eröffnet die Erfindung die Möglichkeit, unter Verwendung ein und derselben Jacke bei zeitlich gegeneinander versetzten Fortbewegungs- beziehungsweise Ganganalysen und/oder für verschiedene in ihrem Körperbau übereinstimmende Individuen Untersuchungsergebnisse zu gewinnen, die miteinander vergleichbar sind.

Besondere Ausführungsarten der Vorrichtung gemäß Patentanspruch 1 und der Jacke gemäß Patentanspruch 12 ergeben sich aus den abhängigen Patentansprüchen 2 bis 11.

Gemäß Patentanspruch 2 weist die erfindungsgemäße Jacke in bevorzugter Ausgestaltung der Erfindung einen Halsteil auf, der an dem mit der Jacke bekleideten Tier den Hals des Tieres umschließt und mit dem oder den Beinteilen und dem Rumpfteil der Jacke einteilig ausgebildet ist und dadurch einen Beitrag zur ortsunveränderlichen Anordnung der Jacke an dem mit der Jacke bekleideten Tier leistet.

Ausweislich Patentanspruch 3 ist in Weiterbildung der Erfindung eine Jacke vorgesehen, an der sowohl Marker zur Detektion durch einen Tracker als auch Sensoren angebracht sind. In diesem Fall besteht die Möglichkeit, mittels des oder der Marker und des zugeordneten Trackers einerseits und mittels des oder der Sensoren andererseits ein und denselben Parameter der Fortbewegung des untersuchten Tieres zu bestimmen und die auf diese Weise getrennt erzielten Ergebnisse zu deren gegenseitiger Verifizierung zu nutzen.

Gemäß Patentanspruch 4 werden im Falle der Erfindung als Sensoren Inertialsensoren üblicher Bauart bevorzugt. Im Falle der Ausführungsform der Erfindung gemäß Patentanspruch 5 werden als Marker optische, insbesondere retroreflektierende Marker in Verbindung mit einer als Tracker vorgesehenen Kameraanordnung verwendet.

Eine für Analysezwecke verwertbare Erfassung der Bewegung eines optischen Markers kann bei entsprechender Ausführung der Erfassungsanordnung, etwa bei Verwendung einfacher Farbmarkierungen als optische Marker, voraussetzen, dass der Marker durch die zugehörige Kameraanordnung unter einem bestimmten Winkel detektiert wird und dass der Marker und die Kameraanordnung dementsprechend eine gegenseitige Sollpositionierung aufweisen. Vor diesem Hintergrund ist in Weiterbildung der Erfindung das Merkmal gemäß Patentanspruch 6 vorgesehen. Aufgrund der seitlichen Abschirmung des oder der Marker ist eine Detektion der Bewegung des oder der Marker durch die Kameraanordnung nur dann möglich, wenn die gegenseitige Position des oder der Marker einerseits und der als Tracker vorgesehenen Kameraanordnung andererseits für die Gewinnung eines für die Bewegungsanalyse brauchbaren Detektionsergebnisses geeignet ist.

Die Patentansprüche 7 und 8 betreffen erfindungsgemäß bevorzugte Möglichkeiten zur konkreten Umsetzung des Erfindungsmerkmals von Patentanspruch 6.

Die Funktionsfähigkeit der erfindungsgemäßen Vorrichtung zur Bewegungserfassung kann eine besondere Ausrichtung des oder Marker und/oder des oder der Sensoren an der Jacke voraussetzen. Für derartige Fälle ist gemäß Patentanspruch 9 in weiterer bevorzugter Ausgestaltung der Erfindung vorgesehen, dass die Jacke und/oder der oder die Marker oder der oder die Sensoren Justiermittel zur Sollausrichtung des oder der Marker und/oder des oder der Sensoren aufweist.

Patentanspruch 10 betrifft eine erfindungsgemäß bevorzugte Möglichkeit zur Fixierung von Sensoren an der für die Bewegungsanalyse verwendeten Jacke. In den anspruchsgemäßen Aufnahmetaschen sind die Sensoren geschützt untergebracht. Ergänzend oder alternativ können die Aufnahmetaschen dazu dienen, die Sensoren mit einer Sollausrichtung an der Jacke anzubringen. Denkbar sind insbesondere eine Außenkontur eines Sensors und eine Innenkontur einer Aufnahmetasche, die derart aufeinander abgestimmt sind, dass ein in die Aufnahmetasche eingeführter Sensor zwangsläufig mit seiner Sollausrichtung an der Jacke angeordnet ist.

Gemäß Patentanspruch 11 kann die erfindungsgemäße Vorrichtung mehrere Jacken unterschiedlicher Passform und/oder unterschiedlicher Größe umfassen. Die in dieser Weise ausgeführte Vorrichtung erlaubt es, Individuen mit unterschiedlichem Körperbau hinsichtlich ihrer Fortbewegung zu analysieren. Der oder die Marker und/oder der oder die Sensoren sind dabei vorzugsweise lösbar an den einzelnen Jacken angebracht. Infolgedessen können ein und dieselben Marker und/oder ein und dieselben Sensoren an sämtlichen Jacken zum Einsatz kommen. Abgesehen von der Passform und/oder der Größe können die Jacken eines Jackensets einheitlich beschaffen sein.

Nachfolgend wird die Erfindung anhand beispielhafter schematischer Darstellungen näher erläutert. Es zeigen:
- Figur 1:: eine Vorrichtung zur Durchführung einer Ganganalyse bei Hunden mit einer einem Hund angelegten und mit optischen Markern und Sensoren versehenen Jacke erster Ausführung,
- Figur 2:: eine mit optischen Markern und Sensoren versehene Jacke zweiter Ausführung zur Durchführung einer Ganganalyse bei Hunden,
- Figur 3:: eine Vorrichtung zur Durchführung einer Ganganalyse bei Hunden mit einer einem Hund angelegten und ausschließlich mit Sensoren versehenen Jacke,
- Figur 4:: eine ausschließlich mit optischen Markern versehene Jacke zur Durchführung einer Ganganalyse bei Hunden und
- Figur 5:: eine an einer Jacke gemäß den Figuren 1 bis 3 angebrachte Aufnahmetasche für einen Sensor.

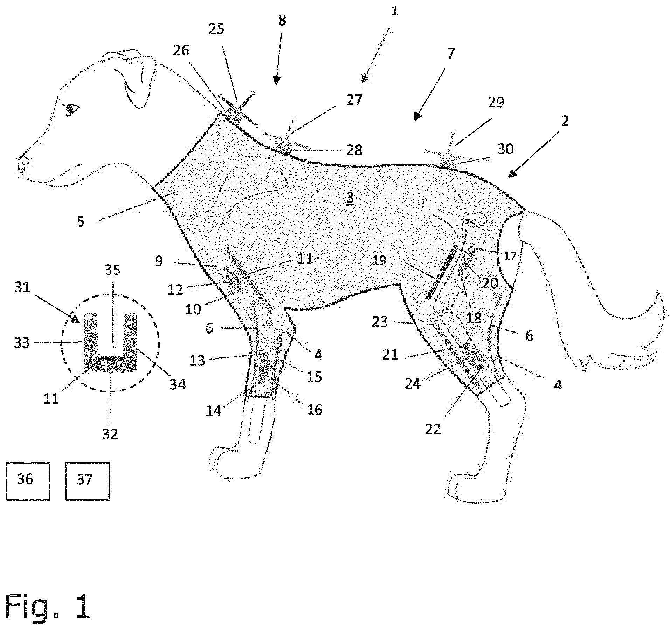

In Figur 1 ist als Vorrichtung zur Bestimmung von Parametern der Fortbewegung von vierbeinigen Säugetieren eine Vorrichtung 1 zur Durchführung einer Ganganalyse bei Hunden dargestellt.

Wesentlicher Bestandteil der Vorrichtung 1 ist eine Jacke 2, die in Figur 1 einem hinsichtlich seines Ganges zu analysierenden Hund angelegt ist.

Die Jacke 2 besteht aus textilem Stretchgewebe und besitzt einen Rumpfteil 3 zur Aufnahme des Rumpfes des Hundes, jeweils einen Beinteil 4 für jede der Extremitäten des Hundes sowie einen Halsteil 5, der den rumpfnahen Teil des Halses des Hundes umschließt. Um das Anlegen der Jacke 2 zu erleichtern, ist jeder der Beinteile 4 mit einem Reißverschluss 6 versehen. In Figur 1 sind die Reißverschlüsse 6 geschlossen. Aufgrund ihrer Passform und aufgrund der Elastizität ihres Materials liegt die Jacke 2 eng und dabei mit einer geringen Vorspannung und gegen ein unerwünschtes Verrutschen gesichert an dem Körper des Hundes an.

Hinsichtlich Passform und/oder Größe ist die Jacke 2 vorkonfektioniert und auf einen üblichen Körperbau von Hunden abgestimmt, die ein bestimmtes Gewicht aufweisen. Weitere, in Figur 1 nicht gezeigte Jacken 2 der Vorrichtung 1 besitzen eine von der dargestellten Jacke 2 verschiedene Passform beziehungsweise Größe und sind für Hunde mit höherem oder niedrigerem Gewicht bestimmt.

An definierten Befestigungspunkten sind an der Jacke 2 als Marker optische Marker 7 und als Sensoren 8 Inertialsensoren angebracht. Die Befestigungspunkte für die optischen Marker 7 und die Sensoren 8 sind derart gewählt, dass die optischen Marker 7 und die Sensoren 8 an dem mit der Jacke 2 bekleideten Hund mit definierter räumlicher Zuordnung zu parameterrelevanten Körperstellen des Hundes angebracht sind.

In dem vorliegenden Beispielsfall ist im Rahmen einer Ganganalyse als Parameter der Fortbewegung des Hundes der Bewegungsbereich ("range of motion") der Beine des Hundes während der Schwingphase des Schrittzyklus zu bestimmen. Für diesen Parameter spezifisch sind die Bewegungen der Ober- und der Unterarme, der Ober- und der Unterschenkel, der Schultern, des Halses und des Beckens des Hundes. Damit ergeben sich die bei der Ganganalyse zu detektierenden parameterrelevanten Körperstellen des Hundes.

An der in Figur 1 sichtbaren linken Körperseite des Hundes sind mittels der Jacke 2 die folgenden optischen Marker 7 und Sensoren 8 mit definierter räumlicher Zuordnung zu den parameterrelevanten Körperstellen des Hundes angebracht:
- auf Höhe des linken Oberarmknochens (Humerus): optische Humerusmarker 9, 10, 11 und ein Humerussensor 12;
- auf Höhe der linken Speiche (Radius): optische Radiusmarker 13, 14, 15 und ein Radiussensor 16;
- auf Höhe des linken Oberschenkelknochens (Femur): optische Femurmarker 17, 18, 19 und ein Femursensor 20;
- auf Höhe des linken Schienbeins (Tibia): optische Tibiamarker 21, 22, 23 und ein Tibiasensor 24.

Entsprechende optische Marker 7 und Sensoren 8 sind an dem der rechten Körperseite des Hundes zugeordneten Teil der Jacke 2 mit definierter räumlicher Zuordnung zu den dortigen parameterrelevanten Körperstellen des Hundes angebracht.

Zusätzlich ist die Jacke 2 am Rücken des Hundes mit folgenden sagittal und dabei mit definierter räumlicher Zuordnung zu den parameterrelevanten Körperstellen des Hundes angebrachten Markern 7 und Sensoren 8 versehen:
- halswärts (zervikal): ein zervikaler optischer Marker 25 und ein zervikaler Sensor 26;
- im Bereich der Schulterblätter (scapular): ein scapularer optischer Marker 27 und ein scapularer Sensor 28;
- im Bereich des Kreuzbeins (sacral): ein sacraler optischer Marker 29 und ein sacraler Sensor 30.

Bei den optischen Humerusmarkern 9, 10, den optischen Radiusmarkern 13, 14, den optischen Femurmarkern 17, 18, den optischen Tibiamarkern 21, 22, dem zervikalen optischen Marker 25, dem scapularen optischen Marker 27 und dem sacralen optischen Marker 29 handelt es sich um sphärische retroreflektierende Marker herkömmlicher Bauart. Als optischer Humerusmarker 11 und auch als optischer Radiusmarker 15, als optischer Femurmarker 19 und als optischer Tibiamarker 23 ist jeweils ein an der Jacke 2 angebrachter reflektierender roter Farbstreifen vorgesehen.

Stellvertretend für die vier letztgenannten Marker zeigt die gestrichelt eingerahmte Teildarstellung von Figur 1 den optischen Humerusmarker 11. Demnach ist der Humerusmarker 11 in ein aus Kunststoff gefertigtes Profil 31 eingebettet, das einen U-förmigen Querschnitt besitzt. Auf einen von einem Quersteg des U-förmigen Querschnitts ausgebildeten Profilboden 32 ist der Humerusmarker 11 aufgeklebt. Eine von Profilseitenwänden 33, 34 begrenzte Profilöffnung 35 weist an der an den Hund angelegten Jacke 2 zu einer in Figur 1 stark schematisch dargestellten Kameraanordnung 36. In entsprechender Weise sind der optische Radiusmarker 15, der optische Femurmarker 19 und der optische Tibiamarker 23 an einem an der Jacke 2 befestigten Profil 31 angebracht.

Die Kameraanordnung 36 ist als Tracker zur Detektion der mit einer Bewegung des Hundes verbundenen Bewegung der optischen Marker 7 und somit zur Detektion der Bewegung der den optischen Markern 7 zugeordneten parameterreleventen Körperstellen des Hundes vorgesehen. In üblicher Weise umfasst die Kameraanordnung 36 mehrere Kameras, die entlang eines von dem Hund bei der Ganganalyse zu passierenden Parcours aufgestellt sind.

Von der Kameraanordnung 36 detektiert wird die Bewegung sämtlicher optischer Marker 7. Eine Detektion des optischen Humerusmarkers 11, des optischen Radiusmarkers 15, des optischen Femurmarkers 19 und des optischen Tibiamarkers 23 durch die Kameraanordnung 36 liefert aber jeweils nur unter der Voraussetzung ein für die Ganganalyse verwertbares Ergebnis, dass die Kameraanordnung 36 den betreffenden optischen Marker 7 unter einem Sollwinkel aufnimmt.

Dass die Detektion des optischen Humerusmarkers 11, des optischen Radiusmarkers 15, des optischen Femurmarkers 19 und des optischen Tibiamarkers 23 durch die Kameraanordnung 36 unter dem Sollwinkel erfolgt, stellen die Profile 31 sicher. Denn nur wenn die Kameraanordnung 36 gegenüber den in Rede stehenden optischen Markern unter dem Sollwinkel ausgerichtet ist, sind der optische Humerusmarker 11, der optische Radiusmarker 15, der optische Femurmarker 19 und der optische Tibiamarker 23 durch die jeweilige Profilöffnung 35 für die Kameraanordnung 36 zur Detektion zugänglich. Andernfalls werden die genannten Marker durch eine der Profilseitenwände 33, 34 des Profils 31 gegen die Kameraanordnung 36 abgeschirmt.

Bei den an der Jacke 2 angebrachten Sensoren 8 handelt es sich um herkömmliche inertiale Messeinheiten (IMU's) mit jeweils drei Translationssensoren und drei gyroskopischen Sensoren. Außerdem denkbar sind beispielsweise als 9d Sensoren ausgebildete Magnetsensoren.

Zur Bestimmung des gesuchten Gangparamaters werden an dem Hund, der den zu diesem Zweck vorgesehenen Parcours abläuft, die mittels der Kameraanordnung 36 und der zugehörigen optischen Marker 7 erfassten Bewegungen der parameterrelevanten Körperstellen des Hundes und die mittels der Sensoren 8 erfassten Bewegungen der parameterrelevanten Körperstellen des Hundes voneinander getrennt in einer hierfür vorgesehenen numerischen Auswerteeinheit 37 rechnergestützt mit Hilfe einer herkömmlichen Software ausgewertet. Die beiden getrennt voneinander ermittelten Ergebnisse der Parameterbestimmung werden zur gegenseitigen Verifizierung genutzt.

Um eine zweckentsprechende Ausrichtung der optischen Marker 7 und der Sensoren 8 an der Jacke 2 zu gewährleisten, sind an der Jacke 2 nicht gezeigte Markierungen vorgesehen, an denen entlang die optischen Marker 7 und die Sensoren 8 auszurichten sind.

Um insbesondere die Sensoren 8 gegen schädliche äußere Einflüsse zu schützen ist es denkbar, dass an der Jacke 2 entsprechende Aufnahmetaschen vorgesehen sind, die zusätzlich für eine Sollausrichtung der Sensoren 8 sorgen können.

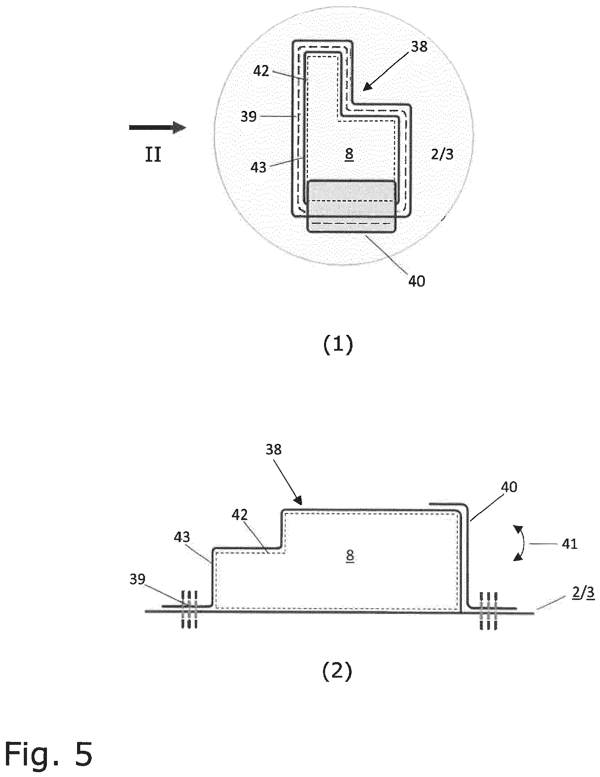

Eine derartige Aufnahmetasche 38 mit einem darin untergebrachten Sensor 8 ist in Figur 5 beispielhaft dargestellt. Teildarstellung (1) von Figur 5 zeigt die Aufnahmetasche 38 mit Sensor 8 in der Draufsicht auf die mit der Aufnahmetasche 38 versehene Jacke 2. In Teildarstellung (2) von Figur 5 ist die Aufnahmetasche 38 mit Sensor 8 in der Ansicht in Richtung des Pfeils II in Teildarstellung (1) gezeigt.

Entlang einer Naht 39 ist die Aufnahmetasche 38 an dem Rumpfteil 3 der Jacke 2 befestigt. Eine Verschlussklappe 40 der Aufnahmetasche 38 kann in Richtung eines Doppelpfeils 41 in eine Offen- oder in die in Figur 5 dargestellte Geschlossenstellung geschwenkt werden. In der Geschlossenstellung ist die Verschlussklappe 40 mittels eines Klettverschlusses an der restlichen Aufnahmetasche 38 fixiert. Die Sollausrichtung des Sensors 8 wird durch das Zusammenspiel einer Außenkontur 42 des Sensors 8 und einer auf die Außenkontur 42 des Sensors 8 abgestimmten Innenkontur 43 der Aufnahmetasche 38 bewirkt. Die Außenkontur 42 des Sensors 8 und die Innenkontur 43 der Aufnahmetasche 38 bilden Justiermittel und sorgen als solche dafür, dass der Sensor 8 nur mit einer Ausrichtung gegenüber der Aufnahmetasche 38 in diese eingeschoben werden kann, aufgrund derer er im eingeschobenen Zustand seine Sollausrichtung aufweist.

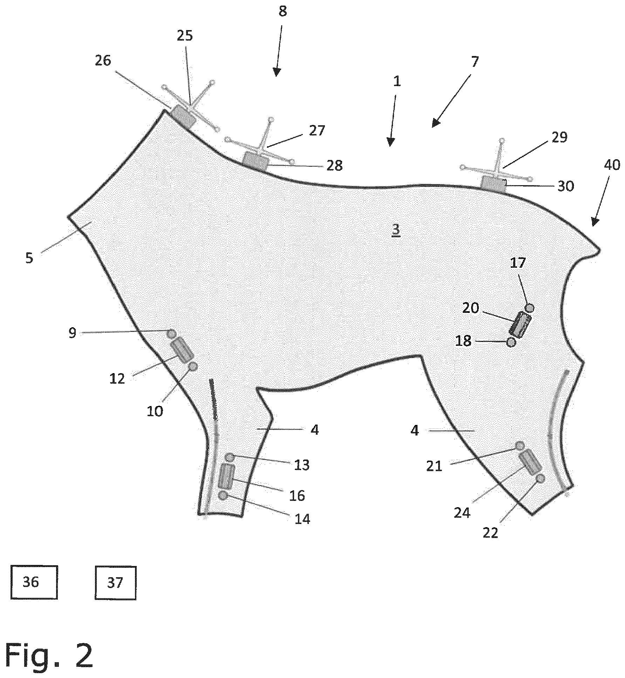

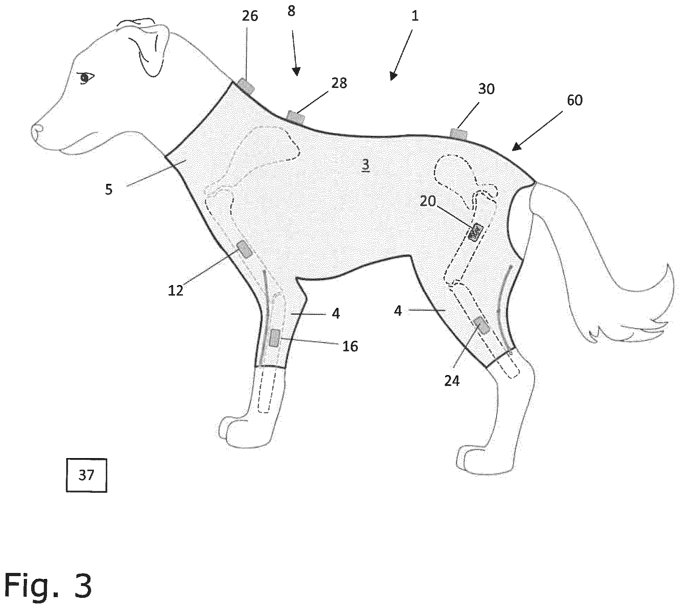

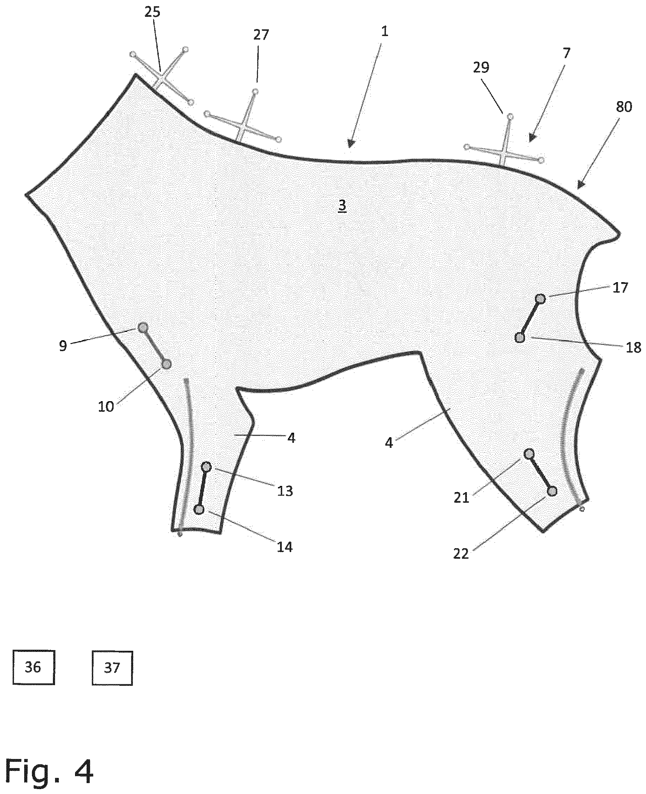

Anstelle der vorstehend im Einzelnen beschriebenen Jacke 2 kann die Vorrichtung 1 zur Durchführung einer Ganganalyse bei Hunden eine Jacke 40 gemäß Figur 2, eine Jacke 60 gemäß Figur 3 oder eine Jacke 80 gemäß Figur 4 umfassen.

Von der in Figur 1 gezeigten Jacke 2 unterscheidet sich die Jacke 40 gemäß Figur 2 lediglich durch das Fehlen des optischen Humerusmarkers 11, des optischen Radiusmarkers 15, des optischen Femurmarkers 19 und des optischen Tibiamarkers 23. Zur Anbringung und Sollausrichtung der Sensoren 8 an der Jacke 40 können in Figur 2 nicht gezeigte Aufnahmetaschen 38 gemäß Figur 5 dienen.

Bei Verwendung der Jacke 60 gemäß Figur 3 erfolgt die Detektion der Bewegung der parameterrelevanten Körperstellen des Hundes gänzlich ohne optische Marker 7 und demnach ausschließlich mittels der auch an der Jacke 2 gemäß Figur 1 vorgesehenen Sensoren 8, die an der Jacke 60 ebenfalls mit definierter räumlicher Zuordnung zu den parameterrelevanten Körperstellen des mit der Jacke 60 bekleideten Hundes angeordnet sind. Im Übrigen stimmt auch die Jacke 60 mit der Jacke 2 gemäß Figur 1 in Aufbau und Funktionsweise überein. Insbesondere kann auch die Jacke 60 mit zur Anbringung und Sollausrichtung von Sensoren 8 dienenden Aufnahmetaschen 38 der in Figur 5 gezeigten Art versehen sein.

An der in Figur 4 dargestellten Jacke 80 sind ausschließlich die sphärisch retroreflektierenden optischen Marker 7 von Figur 1 angebracht, im Einzelnen die optischen Humerusmarker 9, 10, die optischen Radiusmarker 13, 14, die optischen Femurmarker 17, 18, die optischen Tibiamarker 21, 22, der zervikale optische Marker 25, der scapulare optische Marker 27 und der sacrale optische Marker 29. Dementsprechend macht die mit der Jacke 80 versehene Vorrichtung 1 zur Ganganalyse keinen Gebrauch von Sensoren 8. In Figur 1 durch den Humerussensor 12, den Radiussensor 16, den Femursensor 20 und den Tibiasensor 24 verdeckte starre Verbindungen zwischen den optischen Humerusmarkern 9, 10, den optischen Radiusmarkern 13, 14, den optischen Femurmarkern 17, 18 und den optischen Tibiamarkern 21, 22 sind in Figur 4 sichtbar.

## Patentansprüche

1. Vorrichtung zur Bestimmung eines Parameters der Fortbewegung von Tieren, nämlich von vierbeinigen Säugetieren, insbesondere von Hunden, Pferden und/oder Kamelen,
• mit wenigstens einem Marker (7) und einem für den Marker (7) vorgesehenen Tracker (36), wobei der Marker (7) mit einer definierten räumlichen Zuordnung zu einer dem Marker (7) zugeordneten parameterrelevanten Körperstelle des Tieres mit dem Körper des Tieres verbindbar ist und mittels des Markers (7) von dem Tracker (36) eine mit der Fortbewegung des Tieres verbundene Bewegung der dem Marker (7) zugeordneten parameterrelevanten Körperstelle detektierbar ist, wobei die mit der Fortbewegung des Tieres verbundene Bewegung der dem Marker (7) zugeordneten parameterrelevanten Körperstelle für den Parameter der Fortbewegung spezifisch ist und/oder
• mit wenigstens einem Sensor (8), der mit einer definierten räumlichen Zuordnung zu einer dem Sensor (8) zugeordneten parameterrelevanten Körperstelle des Tieres mit dem Körper des Tieres verbindbar ist und mittels dessen eine mit der Fortbewegung des Tieres verbundene Bewegung der dem Sensor (8) zugeordneten parameterrelevanten Körperstelle detektierbar ist, wobei die mit der Fortbewegung des Tieres verbundene Bewegung der dem Sensor (8) zugeordneten parameterrelevanten Körperstelle für den Parameter der Fortbewegung spezifisch ist, sowie
• mit einer Auswerteeinheit (37), mittels derer anhand der von dem Tracker (36) erfassten Bewegung der dem Marker (7) zugeordneten parameterrelevanten Körperstelle und/oder anhand der von dem Sensor (8) erfassten Bewegung der dem Sensor (8) zugeordneten parameterrelevanten Körperstelle der Parameter der Fortbewegung bestimmbar ist,
**dadurch gekennzeichnet, dass**
der Marker (7) und/oder der Sensor (8) mit dem Körper des Tieres verbindbar ist, indem der Marker (7) und/oder der Sensor (8) an einer zum Bekleiden des Tieres vorgesehenen Jacke (2, 40, 60, 80) angebracht ist,
• die wenigstens einen Rumpfteil (3) und einen Beinteil (4) aufweist, wobei der Rumpfteil (3) zur Aufnahme des Rumpfes des Tieres und der Beinteil (4) zur Aufnahme einer Extremität des Tieres ausgebildet sind und
• an welcher der Marker (7) derart angeordnet ist, dass der Marker (7) an dem mit der Jacke (2, 40, 80) bekleideten Tier mit der definierten räumlichen Zuordnung zu der dem Marker (7) zugeordneten parameterrelevanten Körperstelle des Tieres angeordnet ist und/oder
• an welcher der Sensor (8) derart angeordnet ist, dass der Sensor (8) an dem mit der Jacke (2, 40, 60) bekleideten Tier mit der definierten räumlichen Zuordnung zu der dem Sensor (8) zugeordneten parameterrelevanten Körperstelle des Tieres angeordnet ist.

2. Vorrichtung nach Anspruch 1, **dadurch gekennzeichnet, dass** die Jacke (2, 40, 60, 80) einen Halsteil (5) zur wenigstens teilweisen Aufnahme des Halses des Tieres aufweist.

3. Vorrichtung nach einem der vorhergehenden Ansprüche, **dadurch gekennzeichnet,**
• **dass** wenigstens ein Marker (7) und wenigstens ein Sensor (8) vorgesehen sind,
• **dass** der Marker (7) und der Sensor (8) an der Jacke (2, 40) derart angeordnet sind, dass an dem mit der Jacke (2, 40) bekleideten Tier der Marker (7) mit der definierten räumlichen Zuordnung zu der dem Marker (7) zugeordneten parameterrelevanten Körperstelle des Tieres und der Sensor (8) mit der definierten räumlichen Zuordnung zu der dem Sensor (8) zugeordneten parameterrelevanten Körperstelle des Tieres angeordnet sind,
• **dass** die mit der Fortbewegung des Tieres verbundene Bewegung der dem Marker (7) zugeordneten parameterrelevanten Körperstelle und die mit der Fortbewegung des Tieres verbundene Bewegung der dem Sensor (8) zugeordneten parameterrelevanten Körperstelle für ein und denselben Parameter der Fortbewegung spezifisch sind und
• **dass** dieser Parameter der Fortbewegung mittels der Auswerteeinheit (37) sowohl anhand der von dem Tracker (36) detektierten Bewegung der dem Marker (7) zugeordneten parameterrelevanten Körperstelle als auch anhand der von dem Sensor (8) detektierten Bewegung der dem Sensor (8) zugeordneten parameterrelevanten Körperstelle bestimmbar ist.

4. Vorrichtung nach einem der vorhergehenden Ansprüche, **dadurch gekennzeichnet, dass** wenigstens ein Sensor (8) als Inertialsensor ausgebildet ist.

5. Vorrichtung nach einem der vorhergehenden Ansprüche, **dadurch gekennzeichnet, dass** wenigstens ein Marker (7) als optischer Marker ausgebildet ist und dass als Tracker (36) eine Kameraanordnung vorgesehen ist.

6. Vorrichtung nach Anspruch 5, **dadurch gekennzeichnet, dass** der optische Marker (7) eine kameraseitig offene seitliche Abschirmung aufweist, die bei einer gegenseitigen Sollpositionierung des optischen Markers (7) und der Kameraanordnung eine Detektion des optischen Markers (7) durch die Kameraanordnung zulässt und die bei einer von der gegenseitigen Sollpositionierung abweichenden gegenseitigen Fehlpositionierung des optischen Markers (7) und der Kameraanordnung den optischen Marker (7) gegen die Kameraanordnung abschirmt.

7. Vorrichtung nach Anspruch 6, **dadurch gekennzeichnet, dass** die Jacke (2) an ihrer Außenseite ein Profil (31) aufweist, das einen mit dem optischen Marker (7) versehenen Profilboden (32) und eine Profilseitenwand (33, 34) umfasst, welche die kameraseitig offene seitliche Abschirmung für den optischen Marker (7) ausbildet, indem an dem mit der Jacke (2) bekleideten Tier der Profilboden (32) dem Tier zugewandt ist und die Profilseitenwand (33, 34) von dem Profilboden (32) zu der Kameraanordnung hin vorsteht und indem die Profilseitenwand (33, 34) bei einer gegenseitigen Fehlpositionierung des optischen Markers (7) und der Kameraanordnung den optischen Marker (7) gegen die Kameraanordnung abschirmt.

8. Vorrichtung nach Anspruch 7, **dadurch gekennzeichnet, dass** das Profil (31) einen U-förmigen Querschnitt aufweist und dass Schenkel des U-förmigen Querschnitts Profilseitenwände (33, 34) und ein die Schenkel verbindender Quersteg des U-förmigen Querschnitts den Profilboden (32) ausbilden.

9. Vorrichtung nach einem der vorhergehenden Ansprüche, **dadurch gekennzeichnet, dass** an der Jacke (2, 40, 60, 80) und/oder an dem Marker (7) oder dem Sensor (8) Justiermittel (42, 43) vorgesehen sind, mittels derer eine Sollausrichtung des an der Jacke (2, 40, 60, 80) angebrachten Markers (7) und/oder des an der Jacke (2, 40, 60, 80) angebrachten Sensors (8) definierbar ist.

10. Vorrichtung nach einem der vorhergehenden Ansprüche, **dadurch gekennzeichnet, dass** die Jacke (2, 40, 60) mit einer Aufnahmetasche (38) für den Sensor (8) versehen ist.

11. Vorrichtung nach einem der vorhergehenden Ansprüche, **dadurch gekennzeichnet, dass** eine Auswahl von mehreren Jacken (2, 40, 60, 80) vorgesehen ist, die eine unterschiedliche Passform besitzen und/oder die an unterschiedliche Körpergrößen und/oder -gewichte von zu bekleidenden Tieren angepasst sind.

12. Jacke zum Bekleiden eines Tieres, nämlich eines vierbeinigen Säugetieres, insbesondere eines Hundes, eines Pferdes und/oder eines Kamels, die wenigstens einen Rumpfteil (3) und einen Beinteil (4) aufweist, wobei der Rumpfteil (3) zur Aufnahme des Rumpfes des Tieres und der Beinteil (4) zur Aufnahme einer Extremität des Tieres ausgebildet sind,
**dadurch gekennzeichnet,**
**dass** an der Jacke (2, 40, 80) wenigstens ein Marker (7) einer Vorrichtung (1) zur Bestimmung eines Parameters der Fortbewegung des Tieres derart angebracht ist, dass der Marker (7) an dem mit der Jacke (2, 40, 80) bekleideten Tier mit einer definierten räumlichen Zuordnung zu einer dem Marker (7) zugeordneten parameterrelevanten Körperstelle des Tieres angeordnet ist und mittels des Markers (7) von einem Tracker (36) eine mit der Fortbewegung des Tieres verbundene Bewegung der dem Marker (7) zugeordneten parameterrelevanten Körperstelle detektierbar ist, wobei die mit der Fortbewegung des Tieres verbundene Bewegung der dem Marker (7) zugeordneten parameterrelevanten Körperstelle für den Parameter der Fortbewegung des Tieres spezifisch ist und/oder
**dass** an der Jacke (2, 40, 60) wenigstens ein Sensor (8) einer Vorrichtung (1) zur Bestimmung eines Parameters der Fortbewegung des Tieres derart angebracht ist, dass der Sensor (8) an dem mit der Jacke (2, 40, 60) bekleideten Tier mit einer definierten räumlichen Zuordnung zu einer dem Sensor (8) zugeordneten parameterrelevanten Körperstelle des Tieres angeordnet ist und mittels des Sensors (8) eine mit der Fortbewegung des Tieres verbundene Bewegung der dem Sensor (8) zugeordneten parameterrelevanten Körperstelle detektierbar ist, wobei die mit der Fortbewegung des Tieres verbundene Bewegung der dem Sensor (8) zugeordneten parameterrelevanten Körperstelle für den Parameter der Fortbewegung des Tieres spezifisch ist.
